# EUROPÄISCHE PATENTANMELDUNG

(11) **EP 4 371 479 A1**
(43) Veröffentlichungstag der Anmeldung: **22.05.2024**
(21) Anmeldenummer: 23209858.2
(22) Anmeldetag: 14.11.2023
(51) Int. Cl.: A61B 5/11, A61B 5/00

(54) **RUHEMÖBEL UND SENSORANORDNUNG FÜR EIN RUHEMÖBEL**

(30) Priorität: 17.11.2022 DE 202022106456 U
(71) Anmelder: DewertOkin GmbH, 32278 Kirchlengern (DE)
(72) Erfinder: HILLE, Armin, 33659 Bielefeld (DE)
(74) Vertreter: Kleine, Hubertus

(57) **Zusammenfassung**

Die Erfindung betrifft eine Sensoranordnung (10) mit einem Schwingungssensor (15) zur Erfassung von Vibrationen, Bewegung und/oder Schall. Die Sensoranordnung (10) zeichnet sich dadurch aus, dass sie einen weiteren Sensor aufweist, der als Drucksensor ausgebildet ist.

## Beschreibung

Die Erfindung betrifft eine Sensoranordnung für ein Ruhemöbel mit einem Schwingungssensor, der zur Erfassung von physiologischen Parametern eines Nutzes des Ruhemöbels dient. Die Erfindung betrifft weiterhin ein Ruhemöbel mit einer derartigen Sensoranordnung. Als Ruhemöbel sind dabei Sitzmöbel wie Ruhesessel und Liegen oder Betten anzusehen.

Insbesondere im klinischen Bereich sind Überwachungsgeräte bekannt, die Atmung und/oder Herztätigkeit eines Patienten im Schlaf überwachen, um bei bedenklichen Herzfunktions- und Kreislaufparametern eingreifen zu können.

Beispielsweise sind Geräte bekannt, die auf einen Nachtisch gestellt werden, und die Geräusche und/oder Bewegungszustände während des Schlafs mithilfe von Mikrofonen und/oder Kameras erfassen. Aus den erfassten Informationen wird ein Schlafzustand abgeleitet, dessen zeitlicher Verlauf aufgezeichnet wird. Der aufgezeichnete Schlafverlauf kann nachträglich abgerufen und ausgewertet werden. Er kann Aufschluss darüber geben, wie tief und erholsam der Schlaf gewesen ist.

Aus der Druckschrift DE 20 2016 105 634 U1 ist ein Überwachungsgerät bekannt, das eine Sensoranordnung mit einem Sensor zur Erfassung von Vibrationen, Bewegungen und/oder Schall aufweist und eine mit dem Sensor verbundene Auswerteeinheit. Der Sensor ist an einem Rahmen oder unter oder auf einer Matratze eines Betts befestigt und erfasst so Herzschlag und/oder Atmung eines Benutzers des Betts, z.B. eines Patienten.

Insbesondere im Pflege- oder Krankenhausbereich kann es zudem erwünscht sein, dass das Pflegepersonal über ein Aufstehen eines Benutzers des Ruhemöbels informiert wird, beispielsweise um einen möglichen Sturz nach dem Aufstehen verhindern zu können.

Aus der Druckschrift US 5, 780,798 A ist ein Bett bekannt, bei dem unter einer Matratze Sensoren angeordnet sind, die abhängig von einem Gewicht schalten, das auf der Matratze lastet. Die Sensoren sind mit einer Warneinrichtung verbunden, die ein hörbares und/oder sichtbares Alarmsignal abgibt, wenn die Sensoren keine Anwesenheit einer Person im Bett anzeigen, also der Benutzer des Betts dieses verlassen hat.

Wenn eine solche Einrichtung zur Anwesenheitsbestimmung zusätzlich zu einer Sensoranordnung zur Erfassung von physiologischen Parametern eingesetzt wird, resultiert dieses in einem erhöhtem Aufwand für die Montage, insbesondere Verkabelung, der unterschiedlichen Anordnungen und entsprechend auch Pflege und Wartung dieser Anordnungen.

Um dieses Problem zu umgehen, ist gemäß der bereits zuvor genannten Druckschrift DE 20 2016 105 634 U1 die Auswerteeinheit des Schwingungssensors dazu eingerichtet, aus einem fehlenden Signal eine Aussage über die Belegung des Betts anzugeben. Allerdings kann ein Belegungssignal dabei nur mit einer Verzögerung von einigen 10 Sekunden bis zu einer Minute ausgegeben werden, da die Vibrationsdaten, z.B. hervorgerufen durch Atmungsbewegungen, naturgegeben mit einer relativ geringen Wiederholfrequenz detektiert werden. Um Fehlalarme bei dem Belegungssignal zu vermeiden, ist eine lange Zeitspanne vorgesehen, die bei nicht detektiertem Vibrationssignal verstreichen muss, bevor der Belegungsalarm ausgegeben wird.

Es ist daher eine Aufgabe der vorliegenden Erfindung, eine Sensoranordnung der eingangs genannten Art bzw. ein damit ausgestattetes Ruhemöbel zu schaffen, bei denen ein geringer Montage- insbesondere Verkabelungsaufwand gegeben ist und bei denen schnell und zuverlässig ein Verlassen des Ruhemöbels erkannt wird.

Diese Aufgabe wird durch eine Sensoranordnung bzw. ein Ruhemöbel mit den Merkmalen des jeweiligen unabhängigen Anspruchs gelöst. Vorteilhafte Ausgestaltungen und Weiterbildungen sind Gegenstand der abhängigen Ansprüche.

Eine erfindungsgemäße Sensoranordnung der eingangs genannten Art zeichnet sich durch einen weiteren Sensor aus, der als Drucksensor ausgebildet ist. Der Drucksensor reagiert auf einen auf ihn ausgeübten statischen Druck bzw. eine statische (Gewichts-) Kraft und ermöglicht so eine An- bzw. eine Abwesenheitskontrolle einer Person im Ruhemöbel schnell, zuverlässig und unabhängig von einer Auswertung von Schwingungsinformationen. Gegenüber einem reinen Schwingungssensor, dessen Informationen auch zur Bestimmung der An- bzw. Abwesenheit einer Person ausgewertet werden, ergibt sich ein Zeitvorteil und ein höheres Sicherheitsniveau. Gegenüber der Verwendung zweier getrennt voneinander montierter Sensoren vereinfacht die erfindungsgemäße Integration von Schwingungs- und Drucksensor den Montage- und Verkabelungsaufwand im Ruhemöbel.

In einer vorteilhaften Ausgestaltung der Sensoranordnung ist der Schwingungssensor ein piezoelektrischer, elektromagnetischer, elektromechanischen bzw. mikro-elektromechanischer Sensor. Weiter vorteilhaft weist der Drucksensor einen Drucktaster, einen druckabhängigen Widerstand und/oder einen Dehnungsmessstreifen auf. Die genannten Sensoren erlauben jeweils zuverlässige Messungen von Schwingungen bzw. Druck, sind kostengünstig und können platzsparend in die Sensoranordnung eingebaut werden.

Insbesondere können der Schwingungssensor und der Drucksensor übereinander in der Sensoranordnung positioniert sein, so dass ein auf die Anordnung von Schwingungssensor und Drucksensor ausgeübter Druck auf beide Sensoren wirkt.

Bei einer besonders kompakten Sensoranordnung sind der Schwingungssensor und der Drucksensor in einem gemeinsamen Gehäuse angeordnet.

In einer weiteren vorteilhaften Ausgestaltung der Sensoranordnung verlaufen Anschlussleitungen des Schwingungssensors und Anschlussleitungen des Drucksensors in einem gemeinsamen Anschlusskabel der Sensoranordnung. So wird der Montageaufwand im Hinblick auf Verkabelung und Anschluss besonders gering.

Die Erfindung wird nachfolgend anhand eines Ausführungsbeispiels mithilfe von Figuren näher erläutert. Es zeigen:
- Fig. 1: ein erstes Ausführungsbeispiel eines Ruhemöbels mit einem elektromotorischen Möbelantrieb und einer Sensoranordnung; und
- Fig. 2: eine schematische Schnittdarstellung einer Sensoranordnung.

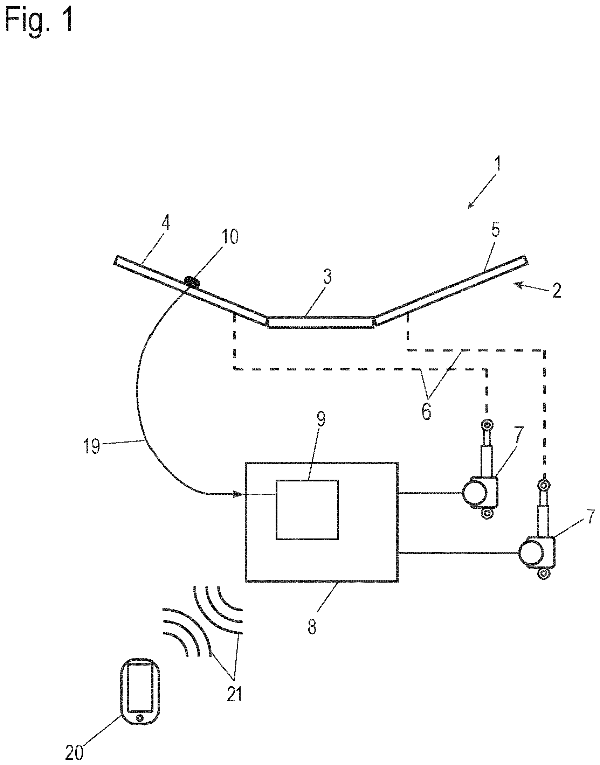

Fig. 1 zeigt ein Bett 1 als Beispiel eines Ruhemöbels mit einer Sensoranordnung 10. Das Bett 1 kann z.B. ein Pflege- oder Klinikbett sein. Es wird angemerkt, dass die Sensoranordnung 10 auch in anderen Ruhemöbeln, z.B. Sesseln, eingesetzt werden kann.

Das Bett 1 weist wenigstens ein Stützelement 2 zur Aufnahme einer hier nicht dargestellten Matratze auf. Das Stützelement 2 ist z.B. aus mehreren plattenförmigen Teilen oder aus einem Lattenrost ausgebildet und auf ein hier nicht wiedergegebenes Grundelement, z.B. ein Fahrgestell, aufgelegt oder montiert.

Das Stützelement 2 weist im dargestellten Beispiel ein Rückenteil 4 und ein Beinteil 5 auf, welche relativ zu einem festen Mittelteil 3 oder relativ zu dem Grundelement beweglich gelagert angeordnet sind. Diese bewegliche Anordnung ist beispielsweise mittels eines so genannten hier nicht dargestellten Bewegungsbeschlags realisiert. Die Bewegung ist verschiebbar und/oder schwenkbar ausgebildet.

Das in diesem Beispiel gezeigte Bett 1 ist mit einer elektromotorischen Verstellanordnung ausgestattet. Das beweglich gelagerte Rückenteil 4 und das Beinteil 5 sind dabei jeweils über eine nur schematisch gezeigte mechanische Verbindung 6 mit je einem elektromotorischen Verstellantrieb 7 gekoppelt.

Die elektromotorischen Verstellantriebe 7 sind vorliegend als Linearantriebe ausgebildet. Die Linearantriebe weisen einen oder eine Anzahl Elektromotoren auf, wobei jedem Elektromotor in der Regel ein Drehzahlreduziergetriebe mit wenigstens einer Getriebestufe nachgeschaltet ist. Dem Drehzahlreduziergetriebe kann ein weiteres Getriebe, beispielsweise in Form eines Gewindespindelgetriebes, nachgeschaltet sein, welches aus der Drehbewegung des Elektromotors eine Linearbewegung eines Abtriebsglieds erzeugt. Das letzte Getriebeglied oder ein damit verbundenes weiteres Glied bildet das Abtriebsglied. Das Abtriebsglied des jeweiligen elektromotorischen Verstellantriebs 7 steht mit dem jeweiligen Möbelbauteil (Rückenteil 4, Beinteil 5) oder alternativ mit einem mit dem Grundelement verbundenen Bauteil in Verbindung, so dass bei einem Betrieb des Elektromotors des jeweiligen Verstellantriebs 7 die beweglichen Möbelbauteile 4, 5 relativ zueinander bzw. relativ zum Grundelement verstellt werden.

Die elektromotorischen Verstellantriebe 7 sind zu ihrer Steuerung elektrisch mit einer Steuereinrichtung 8 verbunden. Diese Verbindung kann z.B. als steckbare Kabelverbindung ausgeführt sein. Die Steuereinrichtung 8 weist eine elektrische Versorgungseinheit auf, welche die elektrische Energie, z.B. aus einem Stromversorgungsnetz, für die elektromotorischen Verstellantriebe 7 bereitstellt. Des Weiteren kann die Kabelverbindung, neben Leitungen zur Energieversorgung der elektromotorischen Verstellantriebe 7, auch Kommunikationsleitungen umfassen, mit welchen die Steuereinrichtung 8 die elektromotorischen Verstellantriebe 7 steuert und/oder deren Betriebsdaten abfragen und einstellen kann.

Zur Energieversorgung der Verstellanordnung kann vorgesehen sein, die Steuereinrichtung 8 über ein hier nicht dargestelltes Netzkabel mit einem Netzanschluss zu verbinden. Das Netzkabel leitet eine eingangsseitige Netzspannung zu der elektrischen Versorgungseinheit der Steuereinrichtung, welche sekundärseitig eine Kleinspannung in Form einer Gleichspannung abgibt. Alternativ hierzu kann der Steuereinrichtung 8 auch eine externe netzabhängige Spannungsversorgungseinheit, auch als Netzteil bezeichnet, vorgeschaltet sein. Das Netzteil hat einen sekundärseitigen Kleinspannungsausgang, welcher über Leitung die Kleinspannung in Form einer Gleichspannung der Steuereinrichtung zuführt.

Die elektrische Versorgung der Verstellanordnung kann auch ausschließlich oder ergänzend zur Netzstromversorgung mittels einer wiederaufladbaren Batterie ("Akku") realisiert sein. Eine solche wiederaufladbare Batterie kann sowohl in der Steuereinrichtung 8, also auch extern davon angeordnet sein und ein integriertes oder externes Ladegerät aufweisen.

Bei der gezeigten Ausgestaltung weist die Steuereinrichtung 8 ein eigenes Gehäuse auf. In alternativen Ausgestaltungen kann vorgesehen sein, die Steuereinrichtung 8 nicht oder nicht vollständig in einem separaten Gehäuse anzuordnen, sondern ganz oder teilweise in einen der Verstellantriebe 7 zu integrieren. Dieser Verstellantrieb stellt dann einen Hauptantrieb dar, an den ggf. weitere Verstellantriebe angeschlossen werden können.

Zur Betätigung der elektromechanischen Verstellantriebe 7 kann eine hier nicht dargestellte Handbedienung vorgesehen sein, die Bedienelemente aufweist, mit denen die Verstellantriebe 7 über die Steuereinrichtung 8 steuerbar sind. Die Handbedienung kann in einem Ausführungsbeispiel über ein Kabel mit der Steuereinrichtung 8 verbunden sein. Alternativ kann die Handbedienung mit einer Übertragungseinrichtung für eine drahtlose Übertragung von Signalen zur Steuereinrichtung 8 versehen sein. Die drahtlose Übertragung kann durch eine Funkübertragungsstrecke, eine optische Übertragungsstrecke (z.B. für Infrarotlicht) und/oder eine Ultraschallübertragungsstrecke realisiert sein, wobei die Steuereinrichtung 8 mit einer jeweiligen entsprechenden Empfangseinheit ausgerüstet ist. Weiter alternativ kann die Handbedienung auch die Steuereinrichtung für die Verstellantriebe bilden, z.B. indem der Betriebsstrom der Verstellantriebe direkt über Schalter der Handbedienung geschaltet wird.

Bei dem dargestellten Bett 1 eine Sensoranordnung 10 vorgesehen, die Vibrationen, Bewegung und/oder Schall detektiert, um physiologische Daten über einen sich im Bett 1 befindenden Person zu erfassen. Die Sensoranordnung 10 ist im dargestellten Ausführungsbeispiel auf einem Rahmenbauteil des Rückenteils 4 befestigt und ist damit unterhalb einer auf der Liegefläche 2 aufgelegten Matratze positioniert. Die Befestigung kann eine Schraub-, Niet- oder Klebeverbindung sein oder auch eine Rast- oder Klemmverbindung, beispielsweise mithilfe einer Federklemme, die das entsprechende Rahmenbauteil umgreift. Die Sensoranordnung 10 weist dazu beispielsweise einen piezoelektrischen, elektromagnetischen und/oder elektromechanischen Sensor auf und ist empfindlich für Schwingungen (Vibrationen) oder Bewegung, die von einer Person im Bett 1 ausgehend und durch die Matratze auf die Sensoranordnung 10 übertragen werden. Ein weiterer geeigneter Sensor ist ein mikro-elektromechanischer Sensor (MEMS-Sensor), z.B. ein mikromechanischer Beschleunigungssensor.

Die genannten Vibrationen umfassen auch Körperschall, der von dem Rückenteil weitergeleitet wird. Unter "Bewegungen" sind insbesondere niederfrequente Schwingungen und dynamische Auslenkungen des Sensors zu verstehen, deren Frequenz im Hertz- oder Sub-Hertz-Bereich liegt. Zusätzlich kann der Sensor empfindlich für (Luft-) Schallwellen sein und in diesem Sinne als ein Mikrofon fungieren.

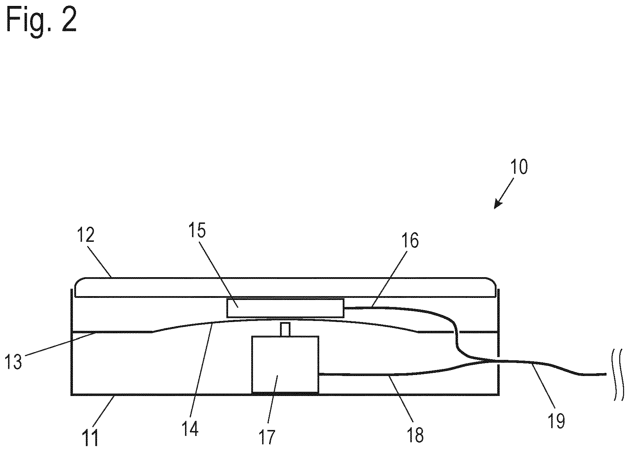

Um eine An- bzw. eine Abwesenheitskontrolle einer Person im Bett 1 schnell und zuverlässig und unabhängig von der Auswertung von Schwingungsinformationen durchführen zu können, ist ein weiterer Sensor in der Sensoranordnung 10 angeordnet. Dieses wird im Zusammenhang mit Fig. 2 weiter unten näher erläutert.

Die Sensoranordnung 10 ist über ein Sensorkabel 19 mit der Steuereinrichtung 8 verbunden. Falls benötigt, wird über das Sensorkabel 19 auch eine Stromversorgung für die Sensoranordnung 10 bereitgestellt und es werden von der Sensoranordnung 10 ausgegebene Signale an die Steuereinrichtung 8 weitergeleitet. In einer alternativen Ausgestaltung kann die Sensoranordnung 10 über eine drahtlose Verbindung, beispielsweise eine Funkverbindung, mit der Steuereinrichtung 8 gekoppelt sein. In dem Fall ist die Sensoranordnung 10 mit einer eigenen Energieversorgung, beispielsweise in Form einer gegebenenfalls wiederaufladbaren Batterie versehen.

Die Steuereinrichtung 8 weist eine Auswerteeinheit 9 zur Verarbeitung und Auswertung der vom Sensor 12 gelieferten Signale auf. Die Auswerteeinheit 9 umfasst z.B. Verstärker und Filtereinheiten, die es ermöglichen, aus der von der Sensoranordnung 10 übermittelten Signale auf bestimmte Körperfunktionen einer sich im Bett 1 befindenden Person zu schließen. Insbesondere ist die Auswerteeinheit dazu eingerichtet, aus den Signalen der Sensoranordnung 10 physiologische Parameter der Person zu ermitteln. Solche Parameter betreffen beispielsweise Herz- und Kreislauffunktionen und umfassen z.B. eine Herzfrequenz und eine Atemfrequenz. Weiter kann ermittelt werden, ob die sich im Bett befindende Person schnarcht. Zudem können Bewegungen der Person erfasst werden.

Die bestimmten physiologischen Parameter werden entweder unmittelbar oder nach Zwischenspeicherung in der Auswerteeinheit 9 bzw. der Steuereinrichtung 8 z.B. mithilfe einer Datenübertragung 21 als drahtlose Signale an ein Mobilgerät 20 als externe Komponente übertragen. Das Mobilgerät 20 kann insbesondere ein handelsübliches Mobiltelefon ("Smartphone") oder ein Tablet-Computer sein und ist mit einer entsprechenden Software ("App") ausgestattet, die eine Auswertung und bevorzugt grafische Darstellung der Zeitabhängigkeit der ermittelten Schlafparameter ermöglicht. Als Übertragungsweg für die Datenübertragung 21 kann beispielsweise WLAN (Wireless Lokal Area Network) oder Bluetooth eingesetzt werden.

Alternativ zur Datenübertragung 21 kann eine indirekte Datenübertragung ausschließlich oder zusätzlich vorliegen, wenn der Datenstrom über andere Komponenten geleitet wird und ggf. dort zwischengespeichert wird. Ein Zwischenspeichern kann temporär erfolgen und nach abgeschlossener Datenübertragung kann ein temporärer Zwischenspeicher wieder gelöscht werden, so dass er für eine nachfolgende weitere Zwischenspeicherung wieder zur Verfügung steht. In einer Ausführung kommt die Cloud als Zwischenspeicher in Betracht. Eine indirekte Datenübertragung ist beispielsweise dann gegeben, wenn das Mobilgerät 20 nach einer Art Modem zum Einsatz kommt, die Daten der Auswerteeinheit mittels einer ersten Übertragungsstrecke, beispielsweise in Form einer Bluetooth-Übertragungsstrecke, aufnimmt und mittels einer weiteren Übertragungsstrecke, beispielsweise WLAN oder Mobilfunk, an eine externe Komponente z.B. in Form der Cloud überträgt.

Zudem kann in der Auswerteeinheit 9 der Steuereinrichtung 8 ein Vergleich der gemessenen physiologischen Parameter mit vorgegebenen Grenzwerten für diese Parameter vorgesehen sein. Werden die ermittelten Parameter unmittelbar, das heißt ohne längere Zwischenspeicherung in der Auswerteeinheit, während der Schlafphase an das Mobilgerät 20 übertragen, kann alternativ oder zusätzlich dort ein solcher Vergleich erfolgen. Bei Über- oder Unterschreiten der Grenzwerte bzw. bei einem Verlassen eines oder mehrerer der Parameter aus einem vorgegebenen Bereich ist vorgesehen, dass die Auswerteeinheit bzw. das Mobilgerät 20 ein Alarmsignal ausgibt. Dieses Alarmsignal kann optisch und/oder akustisch unmittelbar von der Auswerteeinheit und damit z.B. der Steuereinrichtung 8 bzw. dem Mobilgerät 20 ausgegeben werden.

Alternativ oder zusätzlich kann vorgesehen sein, dass das Mobilgerät 20 eine Alarmmeldung über eine weitere, hier nicht dargestellte drahtlose Übertragungsstrecke (z.B. WLAN, Mobilfunknetz) abgibt. Auf diese Weise kann eine weitere Person benachrichtigt werden, falls sich ungewöhnliche Schlafparameter zeigen. Das dargestellte Bett 1 bzw. der elektromotorische Möbelantrieb mit der Sensoranordnung 10 kann so auch zur klinischen Überwachung bzw. zur Patientenüberwachung oder zur Überwachung von Kleinkindern zum Schutz vor plötzlichem Kindstod eingesetzt werden. Beispielsweise kann eine Warn- oder Alarmmeldung abgegeben werden, wenn keine oder als kritisch angesehene physiologische Parameter detektiert werden.

Die Anordnung der Auswerteeinheit 9 in der Steuereinrichtung 8 der elektromotorischen Verstellantriebe 7 ermöglich eine gemeinsame Nutzung von z.B. Netzeilen und Kommunikationsschnittstellen und ist daher vorteilhaft. In alternativen Ausgestaltungen kann die Auswerteeinheit 9 auch separat und unabhängig von der Steuereinrichtung 8 sein. Eine Nutzung des Sensoranordnung kann so auch in einem Ruhemöbel ohne elektromotorische Verstellung erfolgen.

Fig. 2 zeigt eine erfindungsgemäße Sensoranordnung 10, wie beispielsweise beim Bett1 der Fig. 1 eingesetzt, in einer schematischen Schnittdarstellung detaillierter.

Die Sensoranordnung 10 weist ein Gehäuse 11 mit einem beweglich oder elastisch gelagerten Deckel 12 auf. Unterhalb des Deckels 12 ist ein Schwingungssensor 15 angeordnet, der, wie zuvor im Zusammenhang mit Fig. 1 bereits ausgeführt wurde, z.B. als piezoelektrischer, elektromagnetischer, elektromechanischen bzw. mikro-elektromechanischer Sensor ausgebildet sein kann. Schwingungen oder Bewegung, die von einer Person im Bett ausgehend durch die Matratze auf den Deckel 12 übertragen werden, werden von dem Schwingungssensor 15 detektiert.

Um eine An- bzw. eine Abwesenheitskontrolle einer Person im Bett 1 schnell und zuverlässig und unabhängig von der Auswertung von Schwingungsinformationen durchführen zu können, weist die erfindungsgemäße Sensoranordnung 10 neben dem Schwingungssensor 15 einen weiteren Sensor auf, der als Drucksensor ausgebildet ist und der auf einen auf ihn ausgeübten statischen Druck bzw. eine statische (Gewichts-)Kraft reagiert.

Im Gehäuse 11 ist bei der dargestellten Ausgestaltung ein Zwischenboden 13 ausgebildet, der zentral einen elastischen gewölbten Abschnitt 14 aufweist. Der Schwingungssensor 15 ist zwischen dem Deckel 12 und dem gewölbten Abschnitt 14 des Zwischenbodens 13 angeordnet. Statische Druck- bzw. Gewichtskräfte, die auf den Deckel 12 wirken, werden von dem Schwingungssensor 15 auf den gewölbten Abschnitt 14 übertragen, der bei ausreichender Kraft nach unten nachgibt ("umklickt") und damit einen darunter angeordneten Tastschalter 17 betätigt. Der Schaltzustand des Tastschalters 17 gibt somit unmittelbar wieder, ob eine Person im Bett anwesend ist oder nicht.

Die Information über Anwesenheit und insbesondere Abwesenheit der Person kann wie zuvor im Zusammenhang mit den ermittelten physiologischen Parametern erläutert auf verschiedene Weise von der Auswerteeinheit 9 bzw. der Steuereinrichtung 8 in Form einer Warn oder Alarmmeldung ausgegeben werden, z.B. an das Mobilgerät 20

Bei diesem Ausführungsbeispiel stellen der gewölbte Abschnitt 14 des Zwischenbodens 13 zusammen mit dem Tastschalter 17 den Drucksensor dar.

In alternativen Ausgestaltungen kann auch als Drucksensor auch ein druckabhängiger Widerstand (FSR - Force Sensitive Resistor), ein Folientaster oder eine Silikontastermatte eingesetzt werden. Der Drucksensor kann dabei unterhalb oder auch oberhalb des Schwingungssensors 15 angeordnet sein. Weiter kann auch oberhalb oder unterhalb dem Schwingungssensor 15 ein gas- oder flüssigkeitsgefülltes Kissen angeordnet werden, in welchem sich der Druck erhöht, sobald sich eine Person ins Bett legt, wobei der erhöhte Druck im Kissen detektiert wird. Weiter kann auch ein Dehnungsmessstreifen, z.B. auf dem gewölbten Abschnitt 14 des Zwischenbodens 13 angeordnet, als Drucksensor verwendet werden.

Vorteilhaft werden Schwingungsinformationen über den Schwingungssensor 15 und eine An- bzw. Abwesenheitsinformation über den Drucksensor durch die eine Sensoranordnung 10 erfasst. Es muss so nur ein einzelnes Gerät mit einem Gehäuse an dem Bett positioniert werden. Anschlussleitungen 16 des Schwingungssensors 15 und Anschlussleitungen 18 des Tastschalters 17 sind innerhalb des Gehäuses 11 zu dem (einzigen) Anschlusskabel 19 der Sensoranordnung 10 zusammengeführt, was Montage und Kabelführung vereinfacht.

### Bezugszeichenliste

- 1: Bett
- 2: Stützelement
- 3: Mittelteil
- 4: Rückenteil
- 5: Beinteil
- 6: mechanische Verbindung
- 7: Verstellantrieb
- 8: Steuereinrichtung
- 9: Auswerteeinheit

- 10: Sensoranordnung
- 11: Gehäuse
- 12: Deckel
- 13: Zwischenboden
- 14: gewölbter Abschnitt
- 15: Schwingungssensor
- 16: Anschlussleitung
- 17: Tastschalter
- 18: Anschlussleitung
- 19: Anschlusskabel

- 20: Mobilgerät
- 21: Datenübertragung

## Patentansprüche

1. Sensoranordnung (10) mit einem Schwingungssensor (15) zur Erfassung von Vibrationen, Bewegung und/oder Schall, **dadurch gekennzeichnet, dass** die Sensoranordnung (10) einen weiteren Sensor aufweist, der als Drucksensor ausgebildet ist.

2. Sensoranordnung (10) nach Anspruch 1, bei der der Schwingungssensor (15) ein piezoelektrischer, elektromagnetischer, elektromechanischen bzw. mikro-elektromechanischer Sensor ist.

3. Sensoranordnung (10) nach Anspruch 1 oder 2, bei der Drucksensor einen Drucktaster (17) aufweist.

4. Sensoranordnung (10) nach Anspruch 1 oder 2, bei der Drucksensor einen druckabhängigen Widerstand und/oder einen Dehnungsmessstreifen aufweist.

5. Sensoranordnung (10) nach einem der Ansprüche 1 bis 4, bei der der Schwingungssensor (15) und der Drucksensor übereinander angeordnet sind, so das ein auf die Anordnung von Schwingungssensor (15) und Drucksensor ausgeübter Druck auf beide Sensoren wirkt.

6. Sensoranordnung (10) nach einem der Ansprüche 1 bis 5, bei der der Schwingungssensor (15) und der Drucksensor in einem gemeinsamen Gehäuse (11) angeordnet sind.

7. Sensoranordnung (10) nach einem der Ansprüche 1 bis 6, bei der Anschlussleitungen (16) des Schwingungssensors (15) und Anschlussleitungen (18) des Drucksensors in einem gemeinsamen Anschlusskabel (19) der Sensoranordnung (10) verlaufen.
